# EUROPEAN PATENT SPECIFICATION

(11) **EP 2 420 279 B1**
(45) Date of publication and mention of the grant of the patent: **07.01.2015**
(21) Application number: 11445004.2
(22) Date of filing: 16.08.2011
(51) Int. Cl.: A61M 5/32, A61M 5/00

(54) **Cannula needle remover**
Entfernungsvorrichtung für Nadelkanule
Disposition d'extraction d'aiguille de cannule

(30) Priority: 19.08.2010 SE 1000840
(43) Date of publication of application: 22.02.2012
(73) Proprietor: Danderyds Snickeri AB, 187 66 Täby (SE)
(72) Inventor: Hvéren, Lennart, 182 34 Danderyd (SE); Hvéren, Peter, 182 49 Enebyberg (SE)
(74) Representative: Stein, Jan

(56) References cited:
- US-A- 3 444 620
- US-A- 4 531 437
- US-A- 5 588 966
- US-A1- 2003 213 713

## Description

### Technical field of the invention

The present invention is a cannula needle remover that comprises a separation mechanism to separate a cannula needle from a syringe.

### State of the Art

From US 3,444,620 a shearing device is previously known, said device shearing off the cannula from hub portion of a syringe. Thus the hub portion is not removed from the syringe.

From US 4,531,437 a rotary needle and syringe destructor is previously known. Both the cannula and the syringe hub are sheared off from the rest of the syringe.

From US 5,588,966 a device for removing a needle from a syringe is previously known. The fastening part of the cannula is attached to the syringe via a conical seat. The fastening part is removed from the syringe via linear displacement effected by rotatable members equipped with needle removing means.

From US 5,573,113 a cannula needle remover is previously known, that comprises a sleeve which has external spiral grooves. Inside the sleeve is provided a gripping device that cooperates with the cannula needle and prevents rotation of this relative to the sleeve when a syringe is taken into the sleeve. When the syringe is pushed down while rotation is prevented, the sleeve will also be pushed down while it is rotating because of the spiral grooves. Thus, the cannula needle is threaded off from the syringe.

From US 5,067,233 a cannula needle remover is previously known where the cannula needle is brought into the grip using a slit, after which the syringe is rotated until the cannula needle is separated from the syringe.

From US 2003/0029014 a cannula needle remover is previously known that comprises a cannula needle-removing fixture which is rotatably placed in a container. The fixture 28 comprises a cylindrical working part with a hole going through, in which the cannula needle is taken up. Thereafter, the fixture is manually twisted, the cannula needle is deformed and is pulled away from the body of the syringe. See Fig 5 and 6 in US 2003/0029014. When a cannula needle has been removed and has fallen down into the container the fixture/working part must be adjusted so that the through-going hole is back in its original position. It is also necessary for the operator to use both hands while using the cannula needle remover according to US 2003/0029014; one hand holding the syringe and one hand that rotates the working part.

### Objects and Features of the Invention

A primary object of the present invention is to provide a cannula needle remover that is extremely user-friendly, i.e. the user needs to use only one hand.

An additional object of the present invention is that it comprises, in principle, only one moving part, and that the moving part automatically goes back to its original position after use.

Yet another object of the present invention is that the cannula needle remover can handle syringes from different manufacturers.

At least the primary object of the present invention is realised through a device that has the distinguishing features defined in the adhering independent patent claim 1. Preferred embodiments of the invention are defined in the dependent patent claims.

### Brief description of the drawings

Below, a preferred embodiment of the invention will be described, reference being made to the enclosed drawings, where:
- Fig. 1: shows a side view of a cannula needle remover according to the present invention;
- Fig. 1A: shows, using a smaller scale, a perspective view of the cannula needle remover according to Fig. 1;
- Fig. 2: shows a vertical cross-section through the cannula needle remover according to the present invention;
- Fig. 3: shows a vertical cross-section according to the present invention, where a syringe with a cannula needle is taken into the cannula needle remover and is in a starting position;
- Fig. 4: shows a vertical cross-section thought the cannula needle remover according to the present invention, where the cannula needle remover is rotated 90° around an axis in the plane of the paper, compared to Fig. 3;
- Fig. 5: shows a corresponding section through the cannula needle remover as in Fig. 3, however, the syringe has moved to a position where the removal of the cannula needle has commenced;
- Fig. 6: shows a corresponding section through the cannula needle remover as in Fig. 3, where the cannula needle has been separated from the rest of the syringe;
- Fig. 7: shows a corresponding section through the cannula needle remover as in Fig. 3; however, the cannula needle is separated from the rest of the syringe; and
- Fig. 8A-8E: show a number of views and section through a gripping device that is part of the cannula needle remover in the present invention.

### Detailed description of a preferred embodiment of the invention

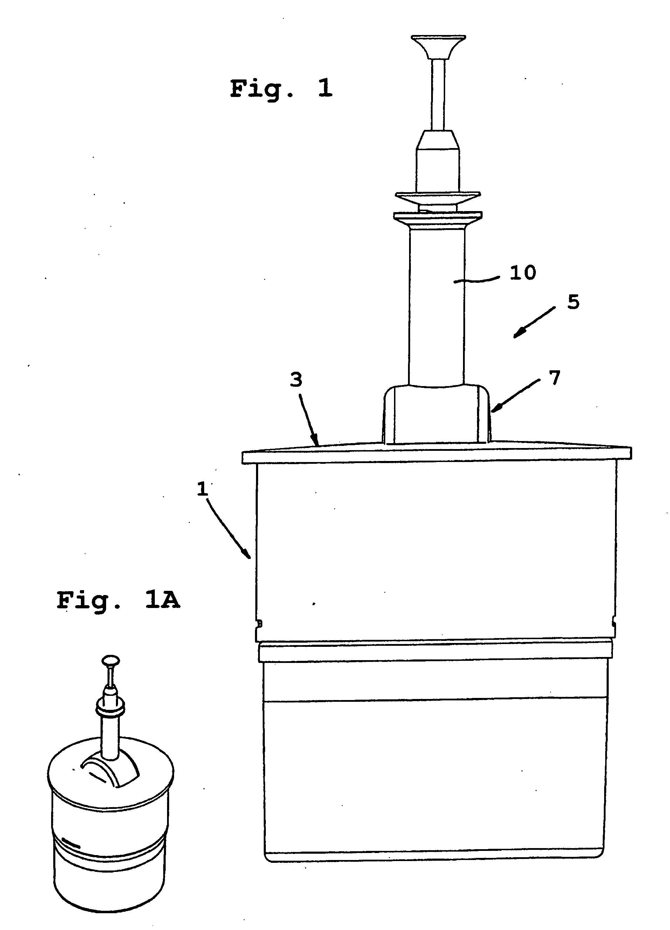

The cannula needle remover shown in Fig. 1 and Fig 1A generally comprises a container 1 open at the top to capture the cannula needles that are removed from a syringe S, as well as a separation mechanism 5 placed in a stationary part 3 of the cannula needle remover. According to the shown embodiment the stationary part 3 is composed of a lid that is applied to the container 1.

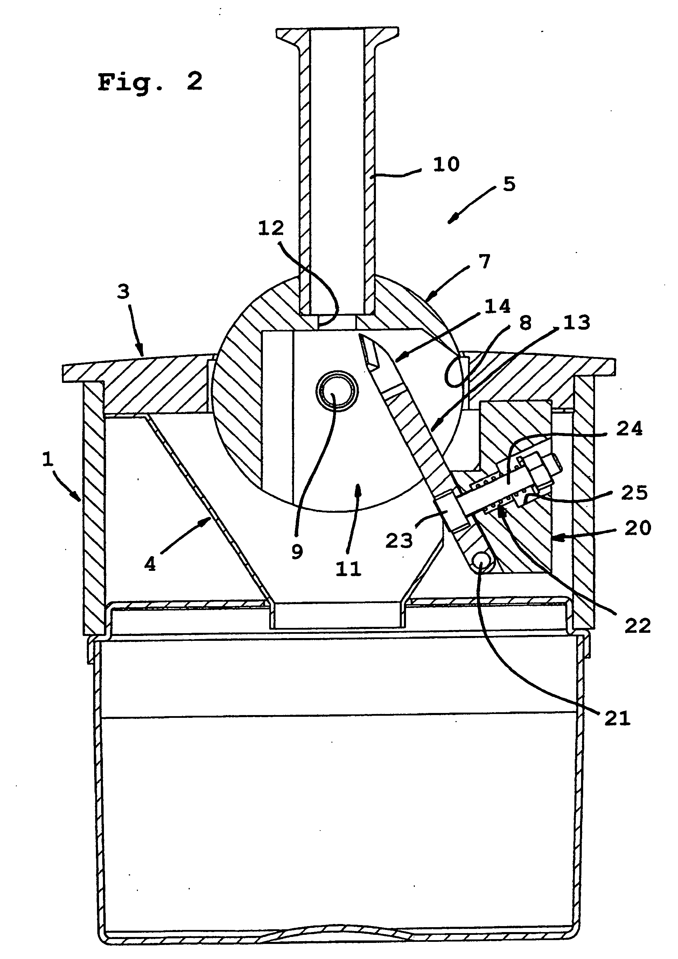

As can be seen from Fig. 2, there is a funnel-like device 4 placed at the bottom side of the lid 2, where the funnel-like device 4 guides down the separated cannula needles K into the container 1.

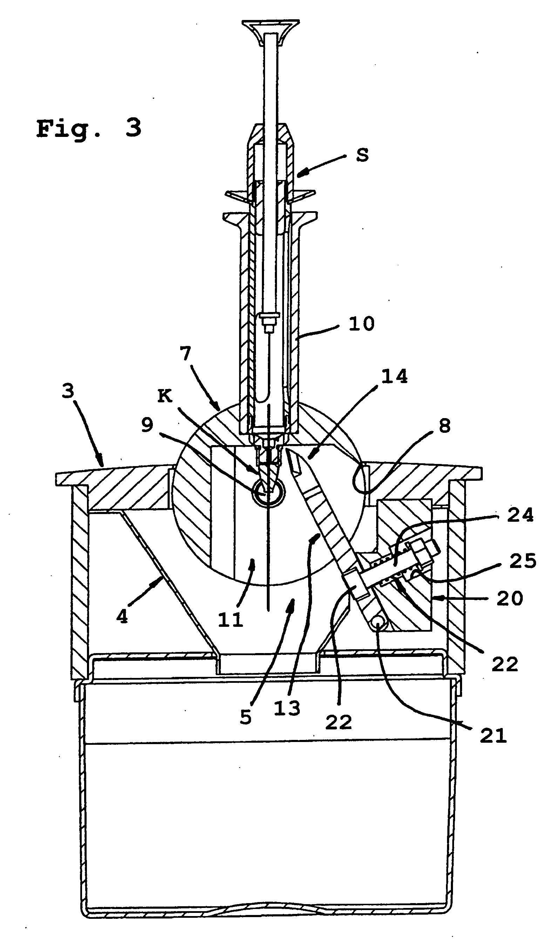

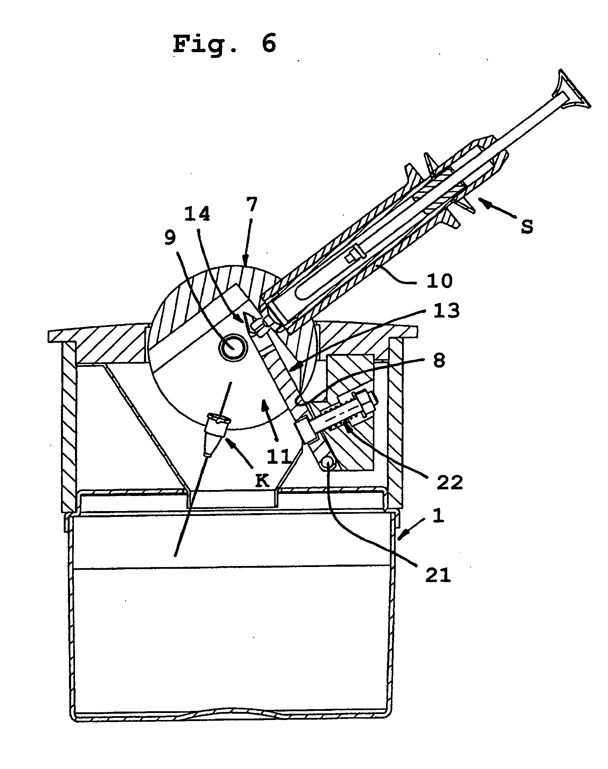

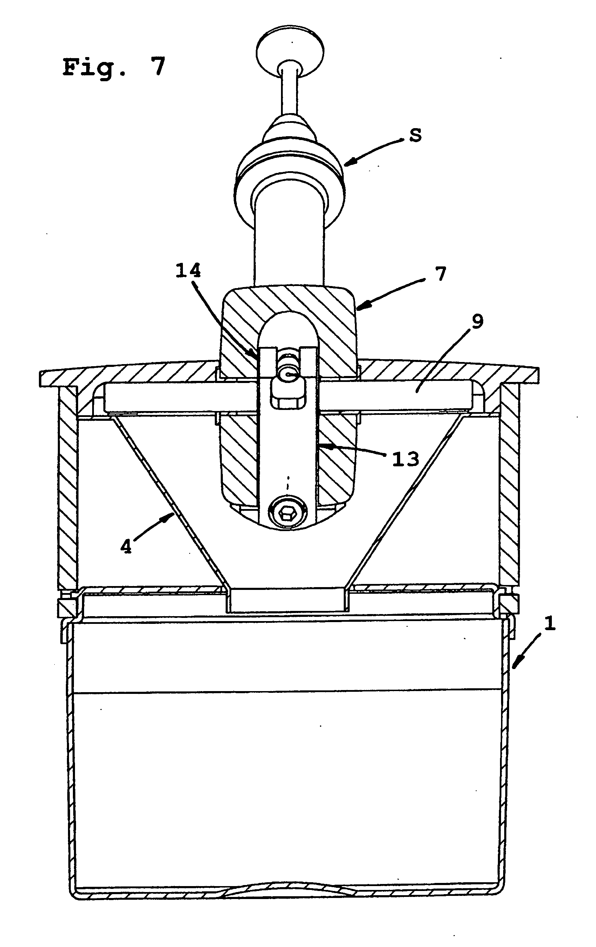

As can be seen most clearly from the Figures 2-7, the separation mechanism 5 comprises a receiving part 7 placed in a first opening in the lid 3, rotatably hanging, which is intended to receive a syringe S whose cannula needle K is to be separated from the rest of the syringe. According to the shown embodiment, the receiving part 7 has generally the shape of a cylinder, where two component axles 9 are part of the separation mechanism 5, and are included in the receiving part 7 and extend axially out from the receiving part 7, on both sides of this part. The ends of the axles 9 are held by bearings in the lid 3, which can be seen most clearly in Fig. 3 and Fig. 6. This arrangement provides that the axles 9 hold the receiving part 7 relative to the lid 3 and that the receiving part 7 can rotate a certain angle relative to the lid 3.

As can be seen generally from Fig. 2-7, a holding device 10 is provided in the receiving part 7, where, according to the shown embodiment, the holding device 10 is in the form of a sleeve that is intended to take up a syringe S that is equipped with a cannula needle K, see e.g. Fig 3. The sleeve is attached to the rest of the receiving part 7 in a fixed fashion. According to the embodiment shown the receiving part 7 is generally cylindrical.

The receiving part 7 has an internally free space 11, where the cannula needle K of the syringe S, received by the receiving part 7, extends into this free space 11. According to the shown embodiment, the free space 11 breaks through the periphery of the receiving part 7 along just less than half of the circumference of the generally cylindrical receiving part 7. The free space 11 is defined by two parallel walls that extend perpendicularly to the axles 9. A stop 8 is defined, where the internal space 11 breaks through the periphery of the receiving part 7 on one of its sides. The lower open end of the holding device 10 is connected to the free space 11 through a second opening 12.

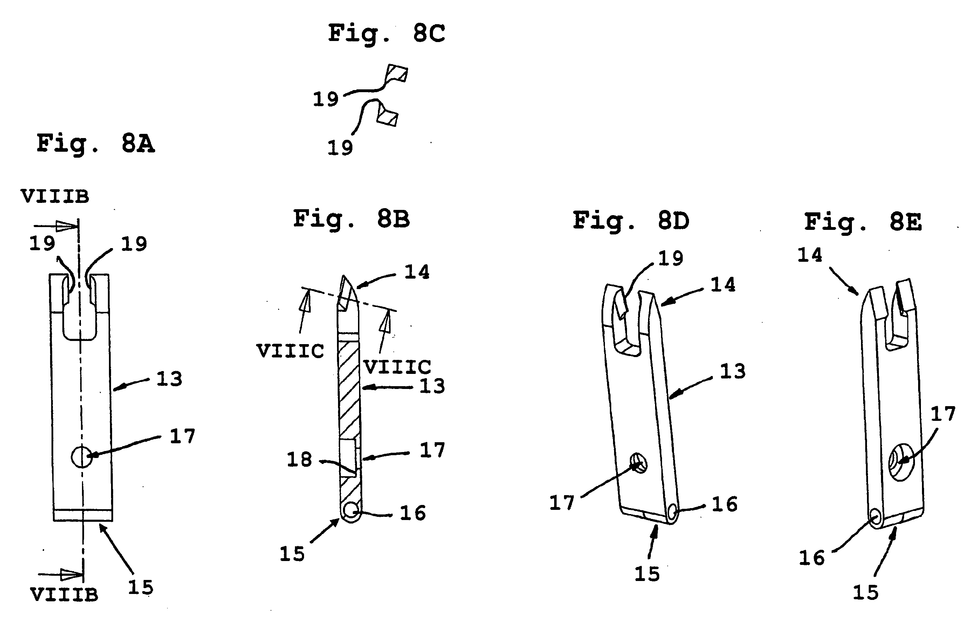

The separation mechanism 5 comprises a gripping device 13 which, according to the embodiment shown, generally has an oblong shape. In Fig. 8A-8E the gripping device 13 is shown separately. The gripping device 13 shows a fork-shaped first end 14 and an opposing second end 15 which is equipped with a first through-going hole 16. In a space in between, the gripping device 13 shows a second through-going hole 17 which is composed of two parts with different diameters, where a shoulder 18 is created in the junction between the two parts with different diameters. The function of second through-going hole 17 will be described below.

The fork-shaped first end 14 of the gripping device shows two opposing edge parts 19, which are parallel and are placed at a distance from each other. The edge parts 19 are relatively sharp, i.e. they are in principle designed as "cutting edges". Preferably, the gripping device 13 is made out of metal, where in particular the edge parts 19 can be made out of tempered steel. According to the lengthwise section in Fig. 8B, through the gripping device 13, the edge parts 19 have a certain inclination relative to the direction along the length of the gripping device 13. The final design of the fork-shaped end 14 has been arrived at empirically.

As can be seen most clearly in Figures 2, 3, 5 and 6, the gripping device 13, through the end 15 which is remote from the fork-shaped end 14, is hingedly connected to a carrying element 20 which is fixedly connected to the stationary part/lid 3. The hinged connection is effected by a pin 21 or similar, that extends through the first through-going hole 16 and a hole in the carrying element 20. To position the gripping device 13 in a flexible fashion in the desired position, a screw 22 is applied in the second through-going hole 17 and the head 23 of the screw 22 abuts the shoulder 18 of the other through-going hole 17. The shaft 24 of the screw 22 extends through a third through-going hole 25 in the carrying element 20, and with an arrangement with a spring, a washer and a nut, the gripping device 13 is made to be in a desired starting position where the gripping device 13 extends in inclined position upwards towards the cannula needle K, where the arrangement with the spring, washer and nut allow that the gripping device can turn around the joint 21 through a limited angle in connection with that a cannula needle K is separated from the syringe S. In this context, it should be noted that the stop 8 at the receiving part 7 is situated in connection with the gripping device 13, that is the stop 8 and the gripping device 13 are situated on the same side of the axles 9.

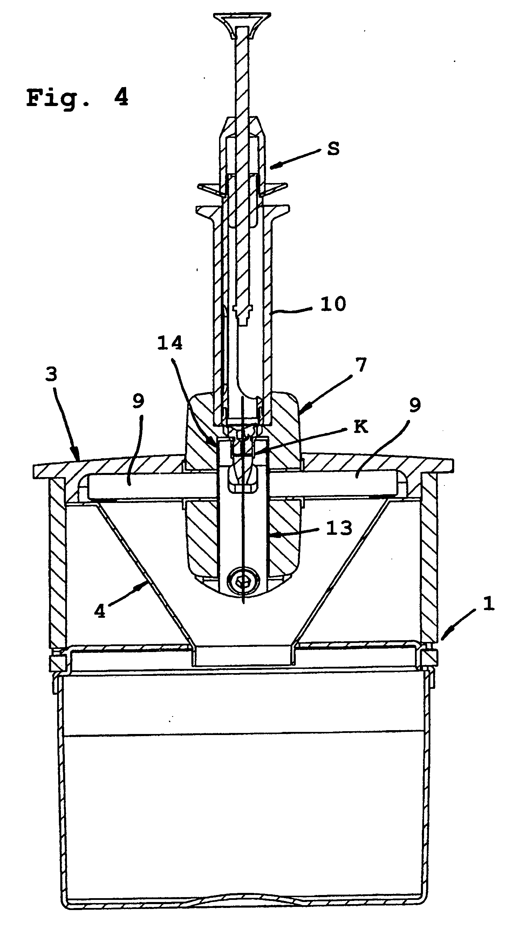

The cannula needle remover according the present invention functions in the following way. The syringe S from which the cannula needle K shall be removed is placed in the holding device 10 of the receiving part 7, where the cannula needle K is taken into the internal space 11 of the receiving part 7. The syringe S at this point will arrive at the position shown in Fig. 3 and Fig. 4. In this context, it should be mentioned that normally, the cannula needle K is connected to the syringe S with a threaded connector.

As can be seen most clearly in Fig. 3, the separation mechanism 5 is also in a starting position, where the receiving part 7 has the holding device 10 pointing upwards and the gripping device 13 at its lower part is in contact with the carrying element 20.

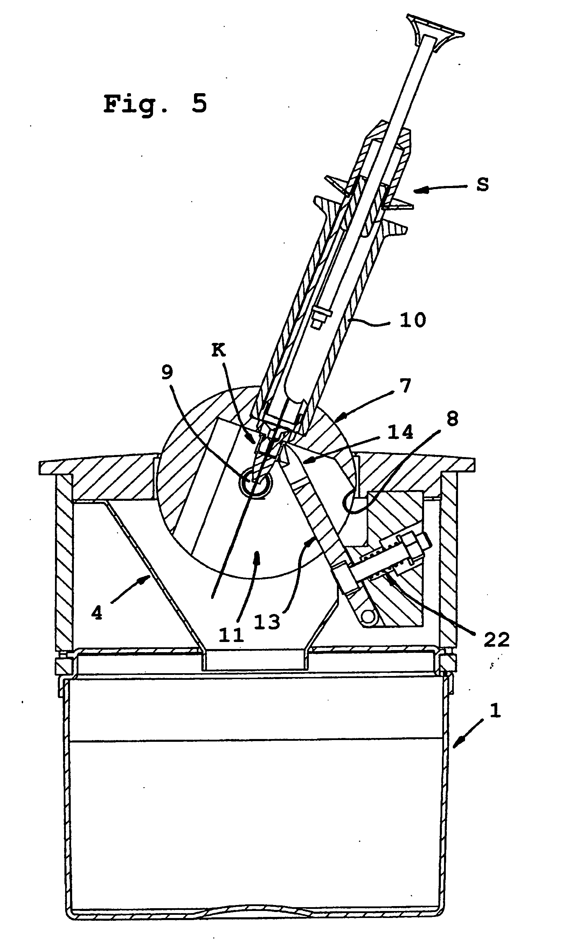

The operator at this point grasps the part of the syringe S protruding from the receiving part 7, and rotates the receiving part 7 from the position shown in Fig. 3, to the position shown in Fig. 5. Thereby the fastening part of the cannula needle K is partly received between the edge parts 19 of the fork-shaped end 14, i.e. the fork-shaped end 14 is in engagement with the fastening part of the cannula needle K. In connection with that the edge parts 19 engage the cannula needle K, the edge parts 19 make a cut into the fastening parts of the cannula needle K. At continuing rotation of the receiving part 7, the stop 8 comes into contact with the gripping device 13 and in the final phase of the rotation of the receiving parts 7 the stop 8 will displace the gripping device 13, which for this reason will turn out slightly from the carrying element 20 around the pin 21. This is illustrated in Fig. 6. This turning outwards of the gripping device 13 causes the fork-shaped end 14 to be displaced away from the syringe S and the cannula needle K is pulled away from the syringe S, at which time the cannula needle K falls down into the container 1. This takes place against the force of the spring. In this context, it should be mentioned that in connection with that the gripping device 13 pulls off the cannula needle K from the syringe S, a certain demolition of the cannula needle K will take place, which results in that it cannot be re-used in an unauthorized way. Normally, there is a demolition of the internal thread that is part of cannula needle K.

When cannula needle K has been removed from the syringe S, the syringe S can be re-used by attaching an unused cannula needle K.

When cannula needle K has been removed from the syringe S, the receiving part 7 is moved back to its starting position, see Fig. 2, and the gripping device 13 returns back to its starting position by the arrangement with spring, washer and nut.

## Claims

1. Cannula needle remover comprising a separation mechanism (5) to separate a cannula needle (K) from a syringe (S), where the separation mechanism (5) comprises a stationary part (3), a receiving part (7) that is, relative to the stationary part (3), to a limited extent rotatable, where the receiving part (7) has a device (10) to receive the end of the syringe (S) that is equipped with the cannula needle (K), where the receiving part (7) has an internal free space (11) in which the cannula needle (K) resides before it is separated from the rest of the syringe, where the separation mechanism (5) comprises a gripping device (13), where a fork-like end (14) of the gripping device (13) is located in the immediate vicinity of the cannula needle (K), where when the receiving part (7) is rotated, the cannula needle (K) will be in engagement with the fork-like end (14), and where there will occur a separation of the cannula needle (K) from the syringe (S) in the final phase of the limited rotation, the fork-like end (14) of the gripping device (13) is provided with opposing edge parts (19) that are in the form of cutting edges, **characterised by** that the gripping device (13) is hingedly connected to the stationary part (3) through a carrying element (20), that the gripping device (13) is spring loaded, that the receiving part (7) is equipped with a stop (8) which is intended to cooperate with the gripping device (13) and that the stop(8) generates a turning of the gripping device (13) at the rotation of the receiving part (7).

2. Cannula needle remover according to claim 1, **characterized by** that the device for taking up the end of the syringe (S) that has the cannula needle (K) is a cylindrical holding device (10) that is fastened to the receiving part (7), and that the holding device (10) communicates with the internal space (11).

3. Cannula needle remover according to claim 1 or 2, **characterized by** that the receiving part (7) is generally cylindrical.

4. Cannula needle remover according to any of the preceding claims, **characterized by** that the receiving part (7) is rotatably held by axles (9) connected via bearings to the stationary part (3).

5. Cannula needle remover according to any of the preceding claims, **characterized by** that the stationary part is in the form of a lid (3) and that the lid is attached to a container (1) for collection of removed cannula needles (K).

## Patentansprüche

1. Entfernungsvorrichtung für Kanülennadel, umfassend einen Trennmechanismus (5) zum Trennen einer Kanülennadel (K) von einer Spritze (S), wobei der Trennmechanismus (5) ein feststehendes Teil (3) und ein Aufnahmeteil (7) umfasst, das in einem eingeschränkten Maße bezogen auf das feststehende Teil (3) drehbar ist, wobei das Aufnahmeteil (7) eine Vorrichtung (10) zum Aufnehmen des Endes der Spritze (S) aufweist, das mit der Kanülennadel (K) ausgestattet ist, wobei das Aufnahmeteil (7) einen inneren Freiraum (11) aufweist, in dem sich die Kanülennadel (K) befindet, bevor sie vom Rest der Spritze getrennt wird, wobei der Trennmechanismus (5) eine Greifvorrichtung (13) umfasst, wobei sich ein gabelähnliches Ende (14) der Greifvorrichtung (13) in der unmittelbaren Nähe der Kanülennadel (K) befindet, wobei, wenn das Aufnahmeteil (7) gedreht wird, die Kanülennadel (K) mit dem gabelähnlichen Ende (14) in Eingriff steht, und wobei eine Trennung der Kanülennadel (K) von der Spritze (S) in der abschließenden Phase der eingeschränkten Drehung stattfindet, das gabelähnliche Ende (14) der Greifvorrichtung (13) mit gegenüberliegenden Randteilen (19) versehen ist, die in Form von Schneidkanten vorliegen, **dadurch gekennzeichnet, dass** die Greifvorrichtung (13) schwenkbar mit dem feststehenden Teil (3) über ein Trageelement (20) verbunden ist, dass die Greifvorrichtung (13) gefedert ist, dass das Aufnahmeteil (7) mit einem Anschlag (8) ausgestattet ist, der mit der Greifvorrichtung (13) zusammenwirken soll, und dass der Anschlag (8) eine Drehung der Greifvorrichtung (13) beim Drehen des Aufnahmeteils (7) erzeugt.

2. Entfernungsvorrichtung für Kanülennadel nach Anspruch 1, **dadurch gekennzeichnet, dass** die Vorrichtung zum Fassen des Endes der Spritze (S), das die Kanülennadel (K) aufweist, eine zylindrische Haltevorrichtung (10) ist, die am Aufnahmeteil (7) befestigt ist, und dass die Haltevorrichtung (10) mit dem Innenraum (11) in Verbindung steht.

3. Entfernungsvorrichtung für Kanülennadel nach Anspruch 1 oder 2, **dadurch gekennzeichnet, dass** das Aufnahmeteil (7) ganz allgemein zylindrisch ist.

4. Entfernungsvorrichtung für Kanülennadel nach einem der vorhergehenden Ansprüche, **dadurch gekennzeichnet, dass** das Aufnahmeteil (7) von Achsen (9), die über Lager mit dem feststehenden Teil (3) verbunden sind, drehbar gehalten ist.

5. Entfernungsvorrichtung für Kanülennadel nach einem der vorhergehenden Ansprüche, **dadurch gekennzeichnet, dass** das feststehende Teil in Form eines Deckels (3) vorliegt und dass der Deckel an einem Behälter (1) zum Sammeln entfernter Kanülennadeln (K) angebracht ist.

## Revendications

1. Dispositif d'extraction d'aiguille de canule, comprenant un mécanisme de séparation (5) destiné à séparer une aiguille de canule (K) d'une seringue (S), le mécanisme de séparation (5) comprenant une partie stationnaire (3), une partie de réception (7) qui est rotative de manière limitée par rapport à la partie stationnaire (3), la partie de réception (7) comportant un dispositif (10) pour recevoir l'extrémité de la seringue (S) qui est équipée de l'aiguille de canule (K), la partie de réception (7) comportant un espace libre intérieur (11) dans lequel l'aiguille de canule (K) se trouve avant d'être séparée du reste de la seringue, le mécanisme de séparation (5) comprenant un dispositif de préhension (13), une extrémité similaire à une fourchette (14) du dispositif de préhension (13) étant située à proximité immédiate de l'aiguille de canule (K), l'aiguille de canule (K) venant en prise avec l'extrémité similaire à une fourchette (14) quand la partie de réception (7) est tournée, et une séparation de l'aiguille de canule (K) et de la seringue (S) se produisant dans la phase finale de la rotation limitée, l'extrémité similaire à une fourchette (14) du dispositif de préhension (13) étant pourvue de parties de bord opposées (19) qui sont sous la forme de bords tranchants, **caractérisé en ce que** le dispositif de préhension (13) est relié de manière articulée à la partie stationnaire (3) par un élément porteur (20), **en ce que** le dispositif de préhension (13) est à ressort, **en ce que** la partie de réception (7) est équipée d'une butée (8) qui est destinée à coopérer avec le dispositif de préhension (13) et **en ce que** la butée (8) génère une rotation du dispositif de préhension (13) lors de la rotation de la partie de réception (7).

2. Dispositif d'extraction d'aiguille de canule selon la revendication 1, **caractérisé en ce que** le dispositif destiné à prendre l'extrémité de la seringue (S) qui comporte l'aiguille de canule (K) est un dispositif de retenue cylindrique (10) qui est fixé à la partie de réception (7) et **en ce que** le dispositif de retenue (10) communique avec l'espace intérieur (11).

3. Dispositif d'extraction d'aiguille de canule selon la revendication 1 ou 2, **caractérisé en ce que** la partie de réception (7) est globalement cylindrique.

4. Dispositif d'extraction d'aiguille de canule selon l'une quelconque des revendications précédentes, **caractérisé en ce que** la partie de réception (7) est maintenue rotative par des axes (9) reliés à la partie stationnaire (3) par le biais de paliers.

5. Dispositif d'extraction d'aiguille de canule selon l'une quelconque des revendications précédentes, **caractérisé en ce que** la partie stationnaire est sous la forme d'un couvercle (3) et **en ce que** le couvercle est fixé à un récipient (1) destiné à recueillir des aiguilles de canule extraites (K).
